(19) Europäisches Patentamt
European Patent Office
Office européen des brevets

(11) Veröffentlichungsnummer: **0 049 853**
**B1**

(12) # EUROPÄISCHE PATENTSCHRIFT

(45) Veröffentlichungstag der Patentschrift: **13.02.85**

(51) Int. Cl.[4]: **C 07 C 177/00,** A 61 K 9/48, A 61 K 31/557

(21) Anmeldenummer: **81107989.6**

(22) Anmeldetag: **06.10.81**

(54) **Neue Prostaglandine, ihre Herstellung und pharmazeutische Kompositionen.**

(30) Priorität: **09.10.80 US 195576**

(43) Veröffentlichungstag der Anmeldung: **21.04.82 Patentblatt 82/16**

(45) Bekanntmachung des Hinweises auf die Patenterteilung: **13.02.85 Patentblatt 85/7**

(84) Benannte Vertragsstaaten: **AT BE CH DE FR GB IT LI LU NL SE**

(56) Entgegenhaltungen: **FR - A - 2 193 613**

(73) Patentinhaber: **F. HOFFMANN-LA ROCHE & CO. Aktiengesellschaft, CH-4002 Basel (CH)**

(72) Erfinder: **Holland, George William, 10 Acorn Place, North Caldwell New Jersey (US)**
Erfinder: **Rosen, Perry, 26 Sunset Drive, North Caldwell New Jersey (US)**
Erfinder: **Gallo-Torres, Hugo, 215 E. Mt.Pleasant Avenue, Livingston New Jersey (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 049 853 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Prostaglandine der Formel

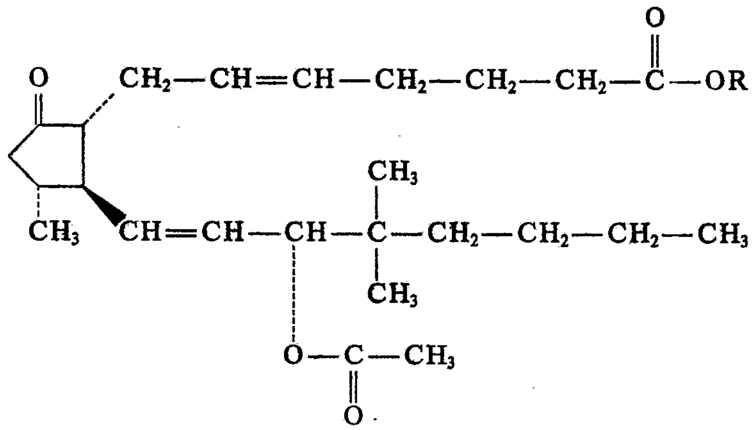

(I)

worin R Wasserstoff oder Niederalkyl ist und pharmazeutisch anwendbare Salze davon.

Die Erfindung betrifft weiterhin ein Verfahren zur Herstellung der Verbindung der Formel I und wenn R = Wasserstoff ist, auch deren pharmazeutisch anwendbaren Salzen, pharmazeutische Kompositionen, die solche Verbindungen enthalten und diese Verbindungen zur Anwendung als Mittel zur Hemmung der Magensaftsekretion.

Der Ausdruck Niederalkyl bezeichnet geradkettige und verzweigte aliphatische Kohlenwasserstoffe mit 1—7 C-Atomen wie Methyl, Äthyl, n-Propyl, Isopropyl und Butyl.

Die Verbindungen der Formel I können erfindungsgemäß aus Verbindungen der Formel

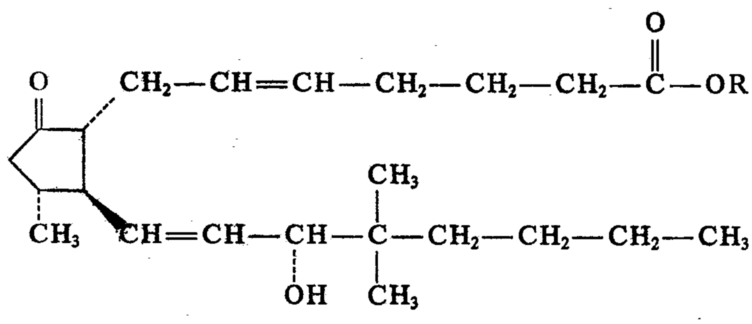

(II)

worin R die obige Bedeutung hat durch Behandlung mit einem Acetylierungsmittel erhalten werden.

Jedes übliche Acetylierungsmittel kann für diese Umwandlung angewandt werden. Beispiele solcher Acetylierungsmittel sind Essigsäure und reaktionsfähige Derivate davon wie Essigsäureanhydrid und Acetylhalogenide. Für die Acetylierung können die üblichen Reaktionsbedingungen Anwendung finden. Temperatur und Druck sind für die Umsetzung nicht kritisch, die Reaktion kann bei Raumtemperatur und Atmosphärendruck durchgeführt werden, andererseits können auch erhöhte oder herabgesetzte Temperaturen angewandt werden. Im allgemeinen ist es bevorzugt die Reaktionen im Temperaturbereich von —10° C bis 50° C durchzuführen.

Die Verbindungen der Formel I sind als antisekretorische Mittel zur Verhütung von Magenhyperazidität von Wert. Die Verbindungen üben einen antisekretorischen Effekt aus, der signifikant länger anhält als bei Verbindungen der Formel II. Dieser Effekt wird durch die Tatsache deutlich gemacht, daß Säurekonzentration und Säureausschüttung im Magen signifikant länger anhaltend reduziert sind, wenn eine Verbindung der Formel I anstelle einer Verbindung der Formel II verabreicht wird.

Die längere Dauer des antisekretorischen Effektes der Verbindung der Formel I bei der Verminderung der Hyperacidität im Magen wird aus einem Vergleich der Resultate der Verabreichung der erfindungsgemäßen Verbindung (8R,11R,12S,15R,5Z,13E)-15-Acetyloxy-11,16,16-trimethyl-9-oxoprosta-5,13,dien-1-säure (Verbindung A) und der entsprechenden 15-Hydroxyverbindung, (8R,11R,12S,15R,5Z,13E)-15-Hydroxy-11,16,16-trimethyl-9-oxoprosta-5,13-dien-1-säure (Verbindung B) deutlich. Dazu wurden diese beiden Verbindungen an der nicht anästhesierten Ratte mit akuter gastrischer Fistel nach dem folgenden Verfahren geprüft:

Am Tag vor dem Experiment wurden gefastete weibliche Ratten (mittleres Gewicht 250 g) chirurgisch kathetisiert in der Vena Cava inferior (zwecks konstanter Infusion von Kochsalzlösung und Verabreichung der Testverbindungen); am Gallengang (um Galle und Pankreassekretion abzuleiten, die durch Rückfluß den Mageninhalt verunreinigen können); am Mageneingang (zwecks Infusion eines kleinen Volumens Wasser während des Experiments); und am Magenausgang (zum Sammeln des Mageninhalts und zur kontinuierlichen Überwachung des pH durch eine Mikroelektrode). Am Tag

des Experiments wurde vor der Verabreichung der Verbindungen der Magen während 60 Minuten mit Wasser gespült. Während dieser Basisperiode betrug das pH (gemessen in 10 Minuten-Intervallen) des Sekretionsflusses bei jedem Tier etwa 1,5. Nach der Basisperiode wurde die zu verabreichende Verbindung, in Polyäthylenglycol-400 gelöst, intragastrisch verabreicht und es wurden für die Dauer von 180 Minuten kontinuierlich Proben gesammelt. Die Proben wurden anschließend untersucht auf Volumen, gesamte Säurekonzentration (µAeq/ml) und gesamte Säureausschüttung während 10 Minuten (µAeq/10 Minuten). Die Resultate sind in Tabelle 1 wiedergegeben.

Tabelle 1

Antisekretorische Aktivität von Prostaglandinen in der wachen Ratte mit akuter gastrischer Fistel

Minuten nach Verabreichung

| Minuten<br>N=<br>Minuten | Verbindung B<br>10 µg/kg Körpergewicht<br>7<br>Konzentration (µAeq/ml)<br>% Hemmung | Verbindung A<br><br>6<br><br>% Hemmung | Verbindung B<br>10 µg/kg Körpergewicht<br>7<br>Ausschüttung (µAeq/10 Minuten)<br>% Hemmung | Verbindung A<br><br>6<br><br>% Hemmung |
|---|---|---|---|---|
| 20 | 62 ( 5)[a] | 34 (16) | 69 ( 3) | 39 (14) |
| 30 | 67 ( 6) | 91 ( 9) | 72 ( 2) | 91 ( 9) |
| 40 | 58 ( 6) | 70 (14) | 68 ( 6) | 69 (17) |
| 50 | 59 ( 5) | 50 (17) | 73 ( 2) | 43 (20) |
| 60 | 43 ( 5) | 67 (21) | 78 ( 7) | 73 (17) |
| 70 | 45 ( 5) | 67 (21) | 71 ( 3) | 74 (17) |
| 80 | 44 ( 6) | 67 (21) | 77 ( 7) | 79 (16) |
| 90 | 25 (13) | 80 (20) | 57 (17) | 80 (20) |
| 100 | | 67 (20) | | 60 (25) |
| 110 | | 60 (24) | | 68 (20) |
| 120 | | 67 (20) | | 72 (20) |
| 130 | | 80 (19) | | 80 (20) |
| 140 | | 100 ( 0) | | 100 ( 0) |
| 150 | | 72 (17) | | 92 ( 5) |
| 160 | | 80 (16) | | 82 (18) |
| 170 | | 80 (16) | | 80 (20) |
| 180 | | 67 (33) | | 71 (29) |

Werte in Klammern beziehen sich auf den Standardfehler.

Zusätzlich zu den intragastrischen Dosierungen wurde die Aktivität der Verbindungen nach intraduodenaler und intravenöser Administration an nicht anästhesierte Ratten mit akuter gastrischer Fistel geprüft. Für die intraduodenale Verabreichung wurden die Verbindungen in Polyäthylenglykol-400 gelöst. Für die intravenöse Verabreichung wurde eine Trimethylol-nitromethan/Äthanol-Lösung verwendet. Die Verbindung A zeigte einen ausgeprägten antisekretorischen Effekt nach intragastrischer Verabreichung in einer Dosis von 10 µg/kg Körpergewicht. Dieser Effekt hielt mindestens 3 Stunden an

und war signifikant länger als der mit der Verbindung B beobachtete. Mit dieser Dosis und Verabreichungsart der Verbindung A wurde ein ausgeprägter Effekt auf das pH beobachtet, nämlich ein mittlerer Anstieg von 3,1 pH-Einheiten im Verlauf von 3 Stunden nach Verabreichung. Mit einer intragastrischen Dosis der Verbindung A von 5 µg/kg Körpergewicht trat ein mittlerer Anstieg von 1,6 pH-Einheiten ein. Dieser Effekt war im wesentlichen äquivalent zu dem, der bei der intragastrischen Verabreichung von 10 µg/kg der Verbindung B auftrat. Die Wirkungsdauer einer intragastrischen Dosis von 5 µg/kg Verbindung A betrug 110 Minuten, diejenige einer Dosis von 10 µg/kg 180 Minuten. Die Beeinflussung des pH mit einer Dosis von 5 µg/kg (etwa 50% von der, die mit einer Dosis von 10 µg/kg Körpergewicht der Verbindung A beobachtet wurde) zeigt deutlich das Vorhandensein einer Dosisabhängigkeit. Der Effekt auf die Ausschüttung von Säure nach intradiodenaler Verabreichung der Verbindung A (10 µg/kg Körpergewicht) war von dem nach intragastrischer Verabreichung der gleichen Verbindung mit der gleichen Dosierung nicht verschieden. Ungeachtet des ausgeprägten Effekts der intraduodenalen Dosierung auf die Ausschüttung von Magensäure war die Wirkung der Verbindung A bei dieser Verabreichungsart auf das pH viel geringer als bei der intragastrischen Verabreichung. Der nach der intravenösen Verabreichung (10 µg/kg Körpergewicht) bei der Verbindung A beobachtete antisekretorische Effekt war niedrig, er betrug etwa $^1/_4$ bis $^1/_5$ der auf intragastrische Verabreichung der gleichen Verbindung beobachteten Wirkung. Bei der intravenösen Verabreichung einer Dosis von 10 µg/kg Körpergewicht der Verbindung A war die Wirkung auf das pH minimal, sie betrug $^1/_{10}$ bis $^1/_{20}$ der nach intragastrischer Verabreichung einer identischen Dosis der gleichen Verbindung beobachteten.

Die Verbindung A wurde weiterhin an wachen Hunden mit Heidenhain — oder Pawlow — Magentasche oder gastrischer Fistel getestet. Bei der oralen Verabreichung der Verbindung A und bei der Verabreichung in die Tasche in Dosen von 10—50 µg/kg und in intravenösen Dosen von 50—100 µg/kg zeigte sich in diesen Modellen bestenfalls, intermittierend, eine schwache bis mäßige Hemmung der Säuresekretion von kurzer Dauer.

Bei der Prüfung der Verbindung A in einer Dosis von 100 µg/kg peroral an 6 Heidenhaim-Hunden zeigte diese Verbindung das Potential für eine signifikante Aktivität. 3 oder 6 untersuchten Hunde zeigten eine Säurereduktion von 50% oder mehr für die Dauer von mehr als 2 Stunden nach der Verabreichung ohne Anzeichen einer Rückkehr zu den Kontrollwerten. Die Verbindung A wurde sodann in einem Dosierungsniveau geprüft, das einen eindeutigen und reproduzierbar pharmakologischen Effekt ergeben sollte. Die gewählte Dosis, 500 µg/kg, bewirkte eine ausgeprägte antisekretorische Aktivität von langer Dauer.

Der Heidenhain-Test besteht darin, daß man Magensaft aus denervierten (Heidenhain) Magentaschen von Bastardhunden sammelt. Dies wird durch Abflußdränage mittels einer besonders hergestellten Titankanüle vorgenommen. Die Magensekretion wurde durch intravenöse Infusion einer Lösung von Histaminhydrochlorid (20 µg/kg/Stunde) in Kochsalzlösung vorgenommen, die mit einer Geschwindigkeit von 1 ml/Minute erfolgte. Diese submaximale Stimulierung provozierte eine Säuresekretion von ungefähr 30% des Maximalwertes, ohne daß die mit höheren Histamindosen häufig beobachtete Tachycardie auftrat. Die Magensaftproben wurden in 15 Minuten-Intervallen gesammelt. Die Verbindung A wurde oral in einer Hartgelatinekapsel (7 g) verabreicht. Die Tiere erhielten das Präparat 90 Minuten nach Beginn der Histamininfusion und der Test wurde für die Dauer von 9 Stunden nach Verabreichung des Medikaments fortgesetzt. Die Hunde waren wach und standen während der Experimente ruhig in Pawlowschlingen. Gemessen wurde das Probenvolumen, das pH, die Säurekonzentration (mAeq/L), Säureausschüttung (mAeq/15 Minuten) sowie Pepsin-Konzentration und -Ausschüttung.

Die zur Beurteilung der Wirksamkeit des Präparates herangezogenen Parameter waren die Säurekonzentration und die Säureausschüttung. Diese Parameter wurden als Prozentsatz des Mittels dreier 15-Minuten-Werte vor der Verabreichung des Präparats ausgedrückt. Die Verabreichung der Verbindung A in einer oralen Dosis von 500 µg/kg bewirkte eine konstante 50%ige oder größere Reduktion der Säurekonzentration der Kontrollwerte (Mittel mg/L) zwischen 4 und $5^3/_4$ Stunden nach Medikation. Diese Dosis der Verbindung A bewirkte weiterhin eine 60%ige Hemmung der Säureausschüttung (Mittel = 0,38 mAeq/15 Minuten) beginnend 45 Minuten nach Verabreichung und während mehr als 9 Stunden. Die Mehrzahl dieser Testperioden zeigte eine 90—98%ige Hemmung der Säureausschüttung. Die pH-Werte nahmen von einem Kontrollmittel von 1,0 auf ein Maximum von 2,0 6 Stunden nach Medikation zu. Die Analyse der Kontrollwerte für diese Gruppe von Versuchstieren zeigte keine signifikante Abnahme der Magensaftvolumina von den Kontrollwerten. Die pH-Werte bei den Kontrolltieren betrugen während des ganzen Experiments im Mittel 0,9. Infolgedessen war keine signifikante Abnahme der Säurekonzentration oder der Säureausschüttung in den Kontrollwerten zu erwarten. Die Auswertung der Daten aus Experimenten, bei denen die Verbindung A in einer oralen Dosis von 250 µg/kg gegeben wurde, zeigte beinahe die gleichgute Aktivität wie die auf dem 500 µg/kg Niveau gefundene.

Zur Untersuchung der Wirkung der Verbindung A auf durch Aspirin bewirkte Magenmikroblutungen wurde eine Technik entwickelt, die auf der von Menasse-Gdynia und Krupp, Toxicol. Appl. Pharmacol. 29, 389—396 verwendeten basierte. Diese Technik besteht darin, daß man Ratten mikrochirurgisch präpariert, um den Magen als zugängliches Perfusionssystem darzustellen, wobei $^{51}$Chrom-markierte

rote Blutzellen ($^{51}$Cr-RBC) als Mittel zur Bestimmung der Blutung von Medikament-induzierten gastrischen Mikroläsionen verwendet wird.

Ratten wurde mikrochirurgisch eine Kanüle in die Vena cava implantiert (zur Injektion von $^{51}$Cr-RBC) sowie eine Kanüle in den Mageneingang (zur konstanten Infusion von Leitungswasser) und eine Kanüle in das Duodenum am Pylorus (zum Sammeln des gesamten Magenausflusses). Nach 30minütigem Waschen mit Leitungswasser erhielt jedes Tier entweder 100 mg/kg Aspirin oder Aspirin zusammen mit der Verbindung A. Jede Dosis wurde intragastrisch in 1 ml 40%igem Polyäthylenglycol-400 als Vehicel verabreicht und wurde 15 Minuten im Magen gelassen. Gleichzeitig wurden 0,9 ml $^{51}$Cr-RBC ($3 \times 10^6$ cpm ≡ Zählimpulse pro Minute) durch die Vena cava verabreicht. Der Magenausfluß wurde alle 5 Minuten im Verlauf von 2 Stunden gesammelt. Die Radioaktivität wurde in einem Gammazähler bestimmt. Die Resultate (Microliter des gesamten im Verlaufe des 60 Minuten langen Experimentes verlorenen Blutes) wurden anhand der folgenden Formel berechnet:

$$\frac{\text{verabreichte Aktivität}}{\text{gesamtes Blutvolumen (ml)}} = \frac{\text{pro Stunde gemessene Aktivität}}{\text{gesamter Blutverlust (ml)}}.$$

Die Resultate zeigten ein Effekt der Verbindung A bei der Verhütung von durch Aspirin verursachte Microblutungen. In einer Dosis von 100 mg/kg verursachte Aspirin alleine ein Mittel von 237 µl Blut in einer Stunde. Wenn die Verbindung A in Kombination mit Aspirin gegeben wurde, wurden die Blutungen in einer dosisabhängigen Weise reduziert oder verhütet. Die entsprechenden Blutwerte nach Verabreichung von 1,25, 2,5 oder 10 µg/kg waren (in µl) 178, 56 und 0.

Die erfindungsgemäßen Verbindungen können wie bereits erwähnt, zur Hemmung der Magensekretion und zur Verhütung von Microblutungen durch orale Verabreichung von Präparaten die eine wirksame Menge einer Verbindung der Formel I oder ein Salz davon enthalten, Verwendung finden. Weiterhin können die Verbindungen der Formel I und deren pharmazeutisch anwendbaren Salze in der Behandlung von Magengeschwüren oder in der Prophylaxe von Magengeschwüren Verwendung finden, insbesondere bei Patienten die an Hyperacidität leiden oder die Stress-induzierter oder chemische Insulte verursachter Geschwürbildung ausgesetzt sind.

Die tägliche Dosierung an Verbindung der Formel I oder eines Salzes davon hängt von den Bedürfnissen des Patienten ab insbesondere dann, wenn ein Geschwür mit Sicherheit festgestellt worden ist. Im allgemeinen beträgt die gesamte tägliche Dosis bei oraler Verabreichung etwa 0,004 mg bis etwa 0,5 mg/kg Körpergewicht. Vorzugsweise beträgt die orale Dosis etwa 0,02 mg bis etwa 0,1 mg/kg Körpergewicht pro Tag. Die Verbindungen der Formel I oder der Salze können enteral mit geeigneten pharmazeutischen Trägern wie Tabletten, Kapseln, Dragées, Sirupen, Suspensionen und Lösungen verabreicht werden. Diese Präparate können auch andere medizinisch wirksame Substanzen sowie auch inerte Bindemittel, Füllstoffe, Träger oder Verdünnungsmittel enthalten. Weiterhin können sie Zusätze wie Geschmacksmittel, Konservierungsmittel, Stabilisatoren, Emulgatoren oder Puffer enthalten. Vorzugsweise enthalten die Präparate ein oder mehrere Antioxidantien wie Ascorbylpalmitat, n-Methyl-α-tocopherolamin, Tocopherole, butyliertes Hydroxyanisol, butyliertes Hydroxytoluol oder Ethoxyquin. Träger und Verdünnungsmittel können organische oder anorganische Substanzen wie Wasser, Gelatine, Lactose, Stärke, Magnesiumstearat, Talk, Gummi arabicum, Polyalkylenglykole sein. Eine bevorzugte systemische Dosierung sind Kapseln aus Hart- oder Weichgelatine, Methylcellulose oder anderem im Verdauungstrakt gleichlöslichen Material.

Die pharmazeutischen Kompositionen enthalten vorzugsweise 0,02 mg bis 10 mg insbesondere 0,3 bis 2 mg Wirkstoff pro Dosierungseinheit.

Erfindungsgemäß können Verbindungen der Formel I mit R Wasserstoff in Form pharmazeutisch anwendbarer Salze mit Basen verwendet werden. Beispiele von solchen Salzen sind Alkalimetallsalze, wie Lithium-, Natrium- und Kaliumsalze, wobei das Natriumsalz bevorzugt ist. Andere Salze sind solche von Erdalkalimetallen wie Calcium und Magnesium, Aminsalze wie Salze von Niederalkylaminen, z. B. Äthylamin und hydroxysubstituierten Niederalkylaminen und Tris(Hydroxymethyl)aminomethan. Ebenfalls bevorzugt sind die Ammoniumsalze. Andere Salze sind solche mit organischen Basen und Aminsalze wie N-Äthylpiperidin, Procain, Dibenzylamin, N-Dibenzyläthyläthylendiamin, Alkylamine oder Dialkylamine und Salze mit Aminosäure, z. B. Salze mit Arginin und Glycin.

Aus FR-A-2 193 613 sind ähnliche Verbindungen bekannt, die jedoch keine 16,16-Dimethylgruppe enthalten. Diese Verbindungen sollen eine Hemmung der Magensäuresekretion bei der Ratte in Dosierungen von 25—100 mg/kg bewirken.

Die folgenden Beispiele erläutern die Erfindung weiter.

## Beispiel 1

Ein Gemisch von 156 mg (8R,11R,12S,15R,5Z,13E)-15-Hydroxy-11,16,16-trimethyl-9-oxoprosta-5,13-dien-1-säure, 100 mg Acetanhydrid, 21 mg 4-(Dimethylamino)pyridin und 2 ml Triäthylamin wurde bei Raumtemperatur unter Stickstoffatmosphäre gerührt. Nach 16 Stunden wurde das Reaktionsgemisch zwischen Äthylacetat und Wasser verteilt, die organische Phase wurde abgetrennt, über Magnesiumsulfat getrocknet und eingedampft. Der Rückstand wurde durch Chromatographie an Sephadex® LH-20 mit Chloroform/Hexan (3 : 2) als Elutionsmittel chromatographiert und lieferte 112 mg (8R,11R,12S,15R,5Z,13E)-15-Acetoxy-11,16,16-trimethyl-9-oxoprosta-5,13-dien-1-säure, $[\alpha]_D^{25}$ −78,25 (Chloroform, c = 1,02).

## Beispiel 2

Nach dem Verfahren von Beispiel 1 wurde aus (8R,11R,12S,15R,5Z-13E)-15-Hydroxy-11,16,16-trimethyl-9-oxoprosta-5,13-dien-1-säuremethylester der (8R,11R,12S,15R,5Z,13E)-15-Acetoxy-11,16,16-trimethyl-9-oxoprosta-5,13-dien-1-säuremethylester erhalten. $[\alpha]_D^{25}$ −95,54 (Chloroform, c = 1,48).

## Beispiel 3

Herstellung von Kapseln:

Kapseln mit folgender Zusammensetzung wurden hergestellt:

| Inhaltsstoffe | mg/Kapsel | | | |
|---|---|---|---|---|
| 1. (8R,11R,12S,15R,5Z,13E)-15-Acetoxy-11,16,16-trimethyl-9-oxoprosta-5,13-dien-1-säure | 0,025 | 0,100 | 0,500 | 1,00 |
| 2. Lactose | 159,975 | 159,90 | 159,50 | 159,00 |
| 3. Modifizierte Stärke | 20,0 | 20,0 | 20,0 | 20,0 |
| 4. Talk | 20,0 | 20,0 | 20,0 | 20,0 |
| Total | 200 mg | 200 mg | 200 mg | 200 mg |

Die Kapseln wurden nach folgendem Verfahren hergestellt:

1. Lösen von 1 in Alkohol.
2. Mischen von 2 und 3, Zugeben der Lösung von 1 über das Gemisch. Trocknen über Nacht.
3. Sieben des Gemisches. Mischen mit Talk.
4. Abfüllen in Kapseln.

In gleicher Weise können Kapseln mit (8R,11R,12S,15R,5Z,13E)-15-Acetoxy-11,16,16-trimethyl-9-oxoprosta-5,13-dien-1-säuremethylester als Inhaltsstoff hergestellt werden.

## Beispiel 4

| | mg/Kapsel | | | |
|---|---|---|---|---|
| (8R,11R,12S,15R,5Z,13E)-Acetoxy-11,16,16-trimethyl-9-oxoprosta-5,13-dien-1-säure | 0,025 | 0,1 | 0,50 | 1,0 |
| Polyäthylenglycol 6000 | 200 | 200,0 | 200,0 | 200,0 |
| Polysorbat 60 | 200 | 200,0 | 200,0 | 200,0 |
| butyliertes Hydroxyanisol | 0,2 | 0,2 | 0,2 | 0,2 |
| Ascorbylpalmitat | 1,0 | 1,0 | 1,0 | 1,0 |

Man erwärmt das Gemisch von Polyäthylenglykol 6000 und Polysorbate 60, gibt das butylierte Hydroxyanisol und das Ascorbylpalmitat dazu, dann das Prostaglandin und löst das Gemisch unter Stickstoff. Danach wird in Hartgelatinekapseln abgefüllt.

Beispiel 5

| | mg/Kapsel | | | |
|---|---|---|---|---|
| (8R,11R,12S,15R,5Z,13E)-Acetoxy-11,16,16-trimethyl-9-oxoprosta-5,13-dien-1-säure | 0,025 | 0,1 | 0,50 | 1,0 |
| Polyäthylenglykol 400 | 100 | 100,0 | 100,0 | 100,0 |
| Polyäthylenglykol 4000 | 300 | 300,0 | 300,0 | 300,0 |
| butyliertes Hydroxyanisol | 0,1 | 0,1 | 0,1 | 0,1 |
| butyliertes Hydroxytoluol | 0,1 | 0,1 | 0,1 | 0,1 |
| Ascorbylpalmitat | 1,0 | 1,0 | 1,0 | 1,0 |

Das Gemisch vonPolyäthylenglykol wird erwärmt, danach wird das butylierte Hydroxytoluol und das Ascorbylpalmitat zugesetzt. Danach das Prostaglandin. Das Gemisch wird unter Stickstoff gelöst und in Hartgelatinekapseln abgefüllt.

**Patentansprüche für die Vertragsstaaten: BE, CH, DE, FR, GB, IT, LI, LU, NL, SE**

1. Verbindungen der Formel

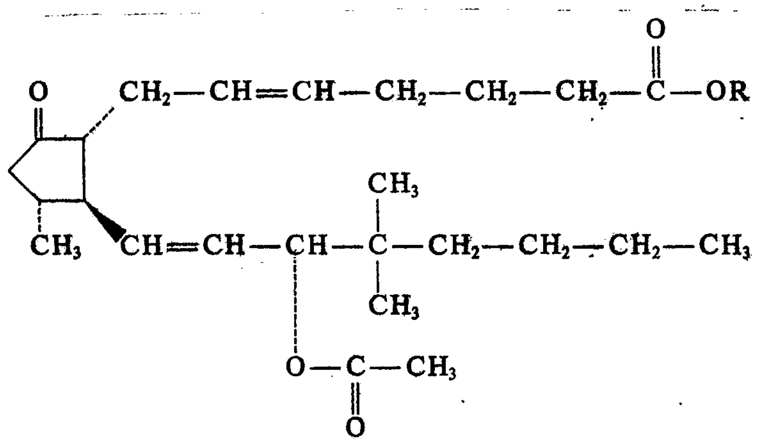

(I)

worin R Wasserstoff oder Niederalkyl ist, und wenn R = Wasserstoff ist, auch pharmazeutisch anwendbare Salze davon.

2. (8R,11R,12S,5Z,13E)-15-Acetoxy-11,16,16-trimethyl-9-oxoprosta-5,13-dien-1-säure.

3. (8R,11R,12S,15R,5Z,13E)-15-Acetoxy-11,16,16-trimethyl-9-oxoprosta-5,13-dien-1-säuremethylester.

4. Eine Verbindung gemäß Anspruch 1 zur Anwendung als Pharmazeutikum.

5. Eine Verbindung gemäß Anspruch 1 zur Anwendung als antisekretorisches Mittel und als Mittel gegen Magengeschwüre.

6. Verfahren zur Herstellung von Verbindungen der Formel 1, dadurch gekennzeichnet, daß man eine Verbindung der Formel

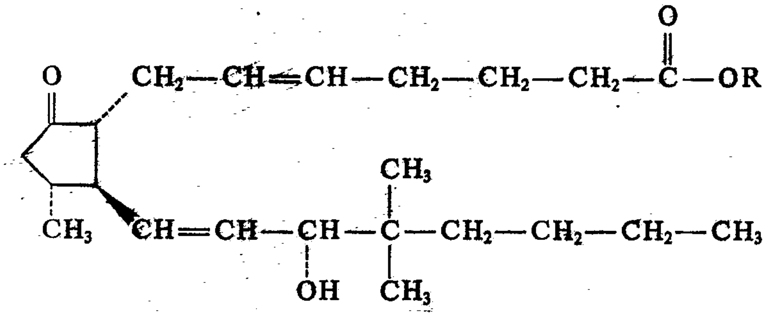

(II)

worin R Wasserstoff oder Niederalkyl ist, mit einem Acetylierungsmittel behandelt.

7. Pharmazeutische Kompositionen, enthaltend eine Verbindung der Formel I von Anspruch 1 oder ein pharmazeutisch annehmbares Salz davon und einen pharmazeutisch anwendbaren Träger.

8. Pharmazeutische Komposition gemäß Anspruch 7, in denen die Verbindung der Formel I oder ein pharmazeutisch anwendbares Salz davon in einer Menge von 0,02 mg bis 10 mg pro Dosierungseinheit enthalten ist.

9. Eine Komposition gemäß Anspruch 8 in Kapselform.

**Patentansprüche für den Vertragsstaat: AT**

1. Verfahren zur Herstellung von Verbindungen der Formel

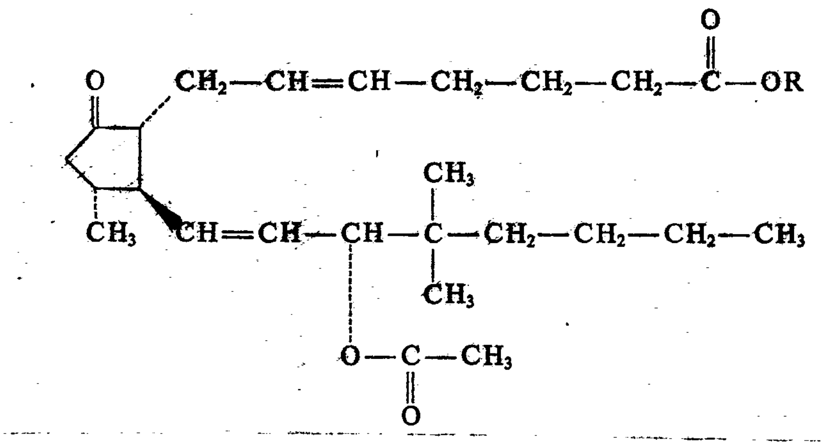

(I)

worin R Wasserstoff oder Niederalkyl ist, und wenn R = Wasserstoff ist, auch von anwendbaren Salzen davon, dadurch gekennzeichnet, daß man eine Verbindung der Formel

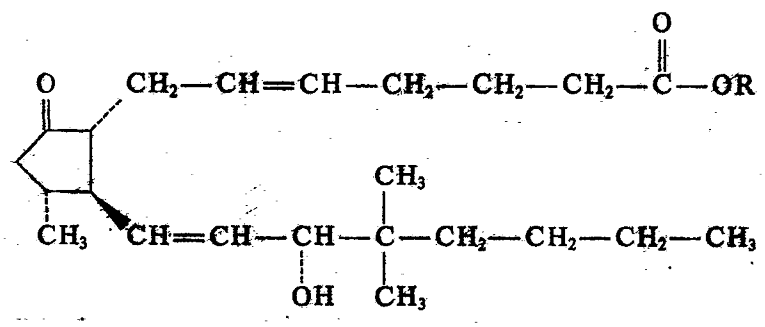

(II)

worin R Wasserstoff oder Niederalkyl ist, mit einem Acetylierungsmittel und gegebenenfalls mit einer Base behandelt.

2. Verfahren nach Anspruch 1, dadurch gekennzeichnet, daß R Wasserstoff ist.

3. Verfahren nach Anspruch 1, dadurch hergestellt, daß R Methyl ist.

**Claims for the Contracting States: BE, CH, DE, FR, GB, IT, LI, LU, NL, SE**

1. Compounds of the formula

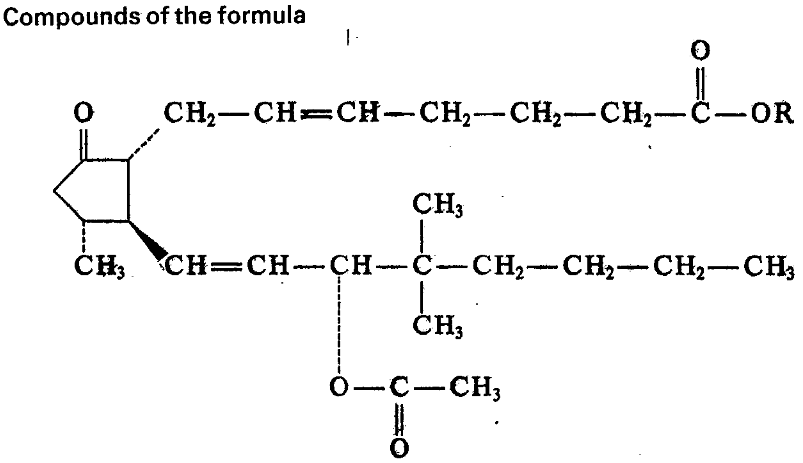

(I)

wherein R is hydrogen or lower alkyl, and, when R is hydrogen, also pharmaceutically usable salts thereof.

2. (8R,11R,12S,5Z,13E)-15-Acetoxy-11,16,16-trimethyl-9-oxoprosta-5,13-dien-1-oic acid.

3. (8R,11R,12S,15R,5Z,13E)-15-Acetoxy-11,16,16-trimethyl-9-oxoprosta-5,13-dien-1-oic acid methyl ester.

4. A compound in accordance with claim 1 for use as pharmaceutical.

5. A compound in accordance with claim 1 for use as an antisecretory agent and as an agent against stomach ulcers.

6. A process for the manufacture of compounds of formula I of claim 1, characterized by treating a compound of the formula

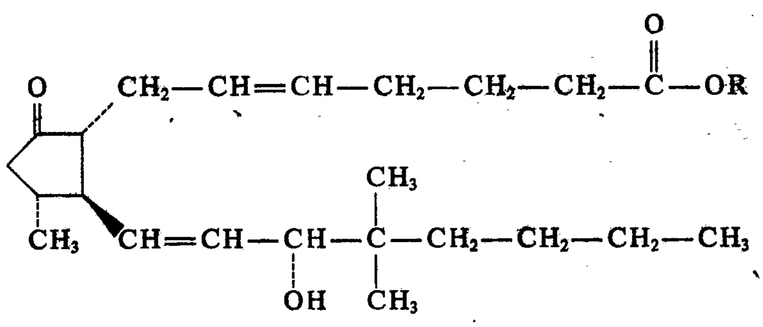

(II)

wherein R is hydrogen or lower alkyl, with an acetylating agent.

7. A pharmaceutical composition, containing a compound of formula I of claim 1 or a pharmaceutically acceptable salt thereof and a pharmaceutically usable carrier.

8. A pharmaceutical composition in accordance with claim 7, in which the compound of formula I or a pharmaceutically usuable salt thereof is present in an amount of 0.02 mg to 10 mg dosage unit.

9. A composition in accordance with claim 8 in capsule form.

**Claims for the Contracting State: AT**

1. A process for the manufacture of compounds of the formula

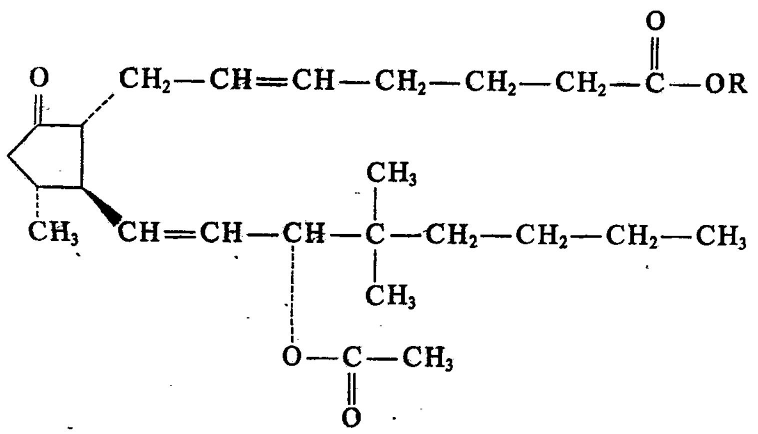

(I)

wherein R is hydrogen or lower alkyl, and, when R is hydrogen, also of pharmaceutically usuable salts thereof, characterized by treating a compound of the formula

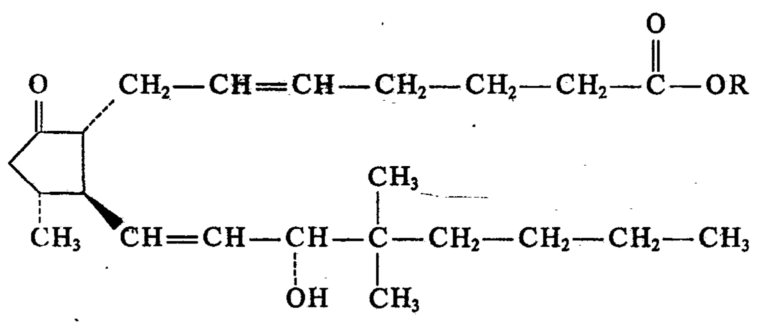

(II)

wherein R is hydrogen or lower alkyl, with an acetylating agent and, if desired, with a base.

2. A process according to claim 1, characterized in that R is hydrogen.

3. A process according to caim 1, characterized in that R is methyl.

**Revendications pour les Etats contractants: BE, CH, DE, FR, GB, IT, LI, LU, NL, SE**

1. Composés de formule:

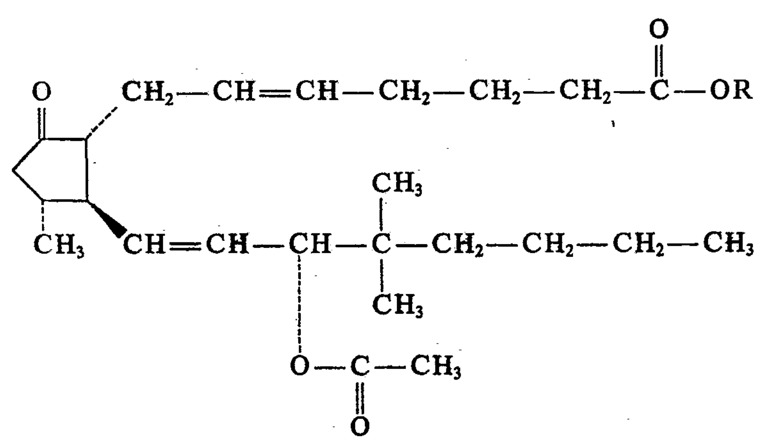

(I)

dans laquelle R représente l'hydrogène ou un groupe alkyle inférieur, et, lorsque R représente l'hydrogène, leurs sels acceptables pour l'usage pharmaceutique.

2. L'acide (8R,11R,12S,15R,5Z,13E) 15-acétoxy-11,16,16-triméthyl-9-oxoprosta-5,13 diène-1-oxïque.

3. Le (8R,11R,12S,15R,5Z,13E)-15-acétoxy-11,16,16-triméthyl-9-oxoprosta-5,13-diène-1-oate de méthyle.

4. Un composé selon la revendication 1 pour l'utilisation en tant que produit pharmaceutique.

5. Un composé selon la revendication 1 pour l'utilisation en tant qu'agent antisécrétoire et en tant

qu'agent protégeant contre les ulcères gastriques.

6. Procédé de préparation des composés de formule 1 selon la revendication 1, caractérisé en ce que l'on traite un composé de formule

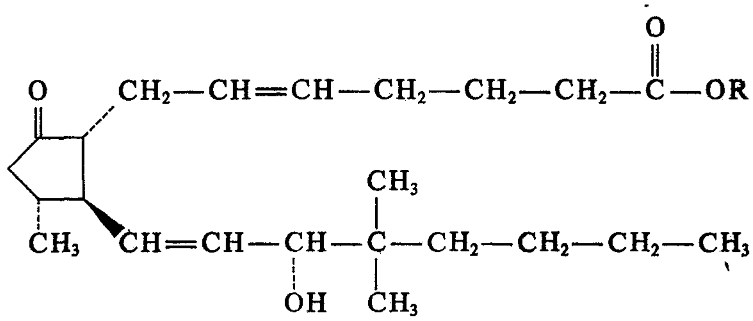

(II)

dans laquelle R représente l'hydrogène ou un groupe alkyle inferieur, par un agent acétylant.

7. Compositions pharmaceutiques contenant un composé de formule 1 selon la revendication 1 ou un sel d'un tel composé acceptable pour l'usage pharmaceutique, et un véhicule acceptable pour l'usage pharmaceutique.

8. Composition pharmaceutique selon la revendication 7, dans laquelle le composé de formule 1 ou un sel d'un tel composé acceptable pour l'usage pharmaceutique est contenu en quantité de 0,02 mg à 10 mg par unité de dosage.

9. Une composition selon la revendication 8 sous forme de capsules.

**Revendications pour l'Etat contractant: AT**

1. Procédé de préparation de composés de formule:

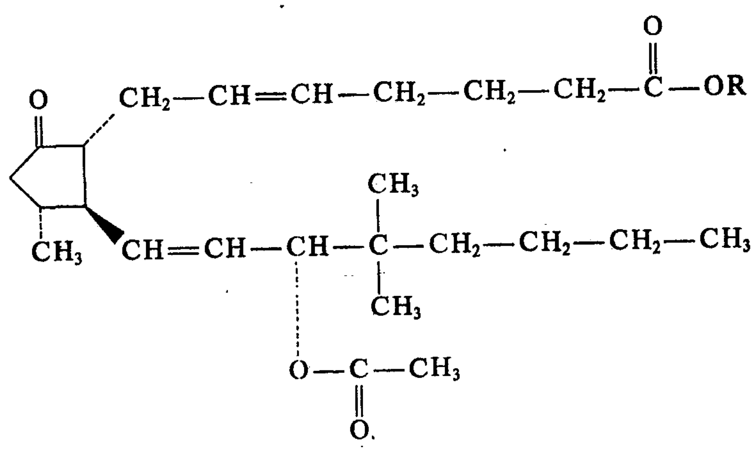

(I)

dans laquelle R représente l'hydrogène ou un groupe alkyle inférieur et, lorsque R représente l'hydrogène, de leurs sels acceptables pour l'usage pharmaceutique, caractérisé en ce que l'on traite un composé de formule:

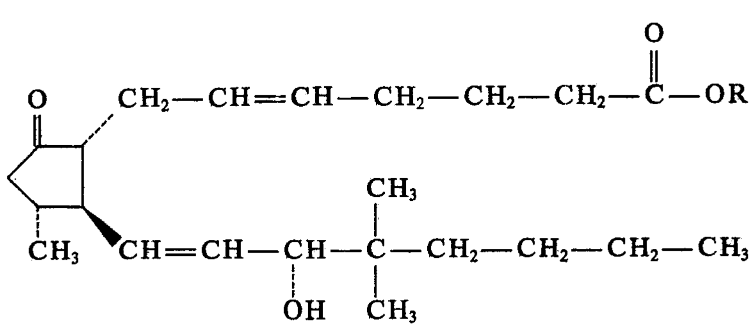

(II)

dans laquelle R représente l'hydrogène ou un groupe alkyle inférieur, par un agent acétylant et le cas échéant une base.

2. Procédé selon la revendication 1, caractérisé en ce que R représente l'hydrogène.

3. Procédé selon la revendication 1, caractérisé en ce que R représente un groupe méthyle.